# EUROPEAN PATENT APPLICATION

(11) **EP 4 810 476 A1**
(43) Date of publication of application: **23.09.2026**
(21) Application number: 24884783.2
(22) Date of filing: 30.10.2024
(51) Int. Cl.: C07C 49/255, C07C 45/81, C07C 229/22, C07C 227/42

(54) **COCRYSTAL OF CURCUMIN AND L-CARNITINE, AND PREPARATION METHOD THEREFOR AND USE THEREOF**

(30) Priority: 02.11.2023 CN 202311456102
(71) Applicant: Cocrystal Health Industry (Zhejiang) Co., Ltd., Jiaxing, Zhejiang 314109 (CN)
(72) Inventor: MEI, Xuefeng, Jiaxing, Zhejiang 314109 (CN); WANG, Hao, Jiaxing, Zhejiang 314109 (CN); ZHANG, Qi, Jiaxing, Zhejiang 314109 (CN); DAI, Wenjuan, Jiaxing, Zhejiang 314109 (CN); LU, Liye, Jiaxing, Zhejiang 314109 (CN)
(74) Representative: GLP S.R.L.
(86) International application number: PCT/CN2024/128358
(87) International publication number: WO 2025/092781

(57) **Abstract**

The present invention relates to a cocrystal of curcumin and L-carnitine, and a preparation method therefor and the use thereof. In the cocrystal of curcumin and L-carnitine of the present invention, the stoichiometric ratio of curcumin to L-carnitine is 1:1, and the cocrystal belongs to a monoclinic system. Compared with curcumin, by means of adding L-carnitine as a ligand to form a cocrystal with curcumin, the present invention changes the intermolecular interaction and spatial arrangement modes of curcumin molecules at the molecular level, which improves the solubility and dissolution performance of curcumin, thereby improving the bioavailability thereof. Furthermore, the method for preparing the cocrystal of curcumin and L-carnitine is simple and easily controllable, and has good reproducibility.

## Description

### Technical Field

The present invention relates to the technical field of pharmaceutical cocrystals, specifically to a cocrystal of curcumin and L-carnitine, a preparation method therefor, products containing said cocrystal, and uses thereof.

### Background Art

Curcumin is a natural polyphenolic compound extracted from the plant Curcuma longa (turmeric). In recent years, a large number of studies have shown that it possesses a wide range of pharmacological activities, including anti-inflammatory, antioxidant, lipid-regulating, antiviral, anti-infective, antitumor, anticoagulant, anti-hepatic fibrosis, and anti-atherosclerotic effects. Curcumin is a natural pigment with bright color, strong coloring power, good antioxidant properties, safety, non-toxicity, and rich nutritional value. It is widely used in various fields such as food, health products, cosmetics, medicine, tobacco, and animal feed. Curcumin is a food additive approved by the FAO/WHO Codex Alimentarius Commission (FAO/WHO-1995) and is one of the earliest natural pigments permitted for use in food according to China's "Hygienic Standards for Uses of Food Additives".

However, curcumin is a fat-soluble pigment. In practical applications, curcumin has some defects, such as poor solubility, low absorption rate, rapid metabolism, and short half-life. These issues lead to low bioavailability, limiting its application in the food and pharmaceutical fields. Current methods to improve the solubility and bioavailability of curcumin primarily involve preparing new drug formulations, such as microemulsions, microspheres, solid dispersions, liposomes, phospholipid complexes, micellar nanoparticles, cyclodextrin inclusion complexes, and dripping pills. However, microspheres, nanoparticles, solid dispersions, and liposomes require large amounts of carrier excipients; microemulsion formulations contain large amounts of surfactants with potential toxicity; preparing phospholipid complexes requires composite reaction between the drug and phospholipids at a certain temperature followed by solvent removal, which may cause degradation of curcumin. Therefore, there is a continuous pursuit for a curcumin product that is convenient to use, high in content, chemically stable, easy to prepare, and low in cost.

### Summary of the Invention

To improve the absorption and bioavailability of curcumin products, the present invention uses edible L-carnitine as a ligand to form a stable cocrystal with curcumin, thereby changing the intermolecular interactions and spatial arrangement of curcumin molecules at the molecular level, increasing the solubility and dissolution performance of curcumin, and thus improving its bioavailability.

In view of this, one objective of the present invention is to provide a cocrystal of curcumin and L-carnitine.

A second objective of the present invention is to provide a method for preparing said cocrystal of curcumin and L-carnitine.

A third objective of the present invention is to provide a product comprising the above-mentioned cocrystal of curcumin and L-carnitine, wherein the product is selected from health products, foods, cosmetics, pharmaceuticals, pharmaceutical excipients, and feeds.

A fourth objective of the present invention is to provide a use of the above-mentioned cocrystal of curcumin and L-carnitine in the preparation of products, wherein the product is selected from health products, foods, cosmetics, pharmaceuticals, pharmaceutical excipients, and feeds.

To achieve the above objectives, the present invention provides the following technical solutions.

In a first aspect, the present invention provides a cocrystal of curcumin and L-carnitine (curcumin-L-carnitine cocrystal), wherein the stoichiometric ratio of curcumin to L-carnitine in the cocrystal is 1:1.

The curcumin-L-carnitine cocrystal belongs to the monoclinic crystal system, with unit cell parameters of a = 17.1370(11) Å, b = 5.6406(4) Å, c = 27.393(2) Å, αbelongs to the mo63(2)b, elongs .

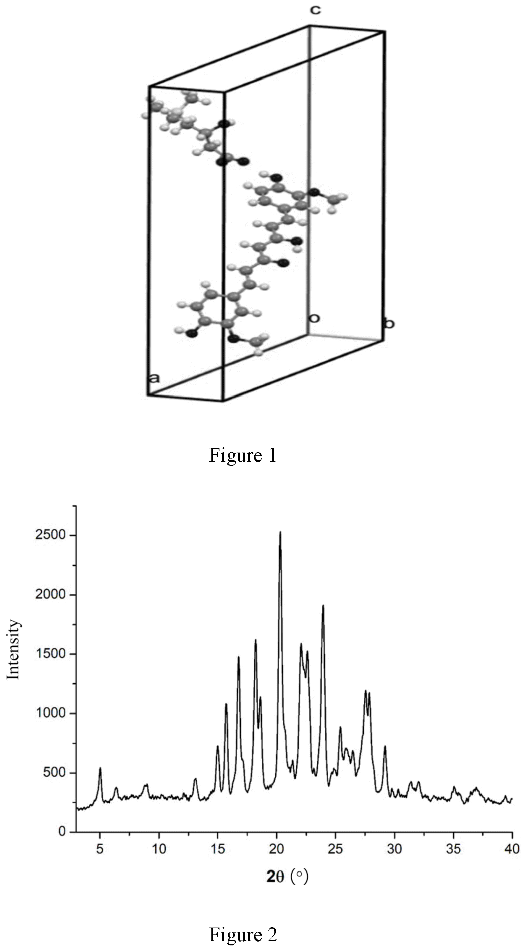

In some embodiments, the X-ray powder diffraction pattern of the curcumin-L-carnitine cocrystal has characteristic peaks at 20 angles of 5.0°±0.2°, 6.4°±0.2°, 13.1°±0.2°, 16.8°±0.2°, 20.3°±0.2°, and 23.9°±0.2°; in particular, it further has characteristic peaks at 20 angles of 18.2°±0.2°, 18.6°±0.2°, 22.1°±0.2°, and 22.6°±0.2°; more particularly, it further has characteristic peaks at 2θ angles of 9.0°±0.2°, 15.0°±0.2°, 15.7°±0.2°, 18.2°±0.2°, 18.6°±0.2°, 22.1°±0.2°, 22.6°±0.2°, and 27.9°±0.2°; preferably, the curcumin-L-carnitine cocrystal has an X-ray powder diffraction pattern substantially as shown in Figure 2.

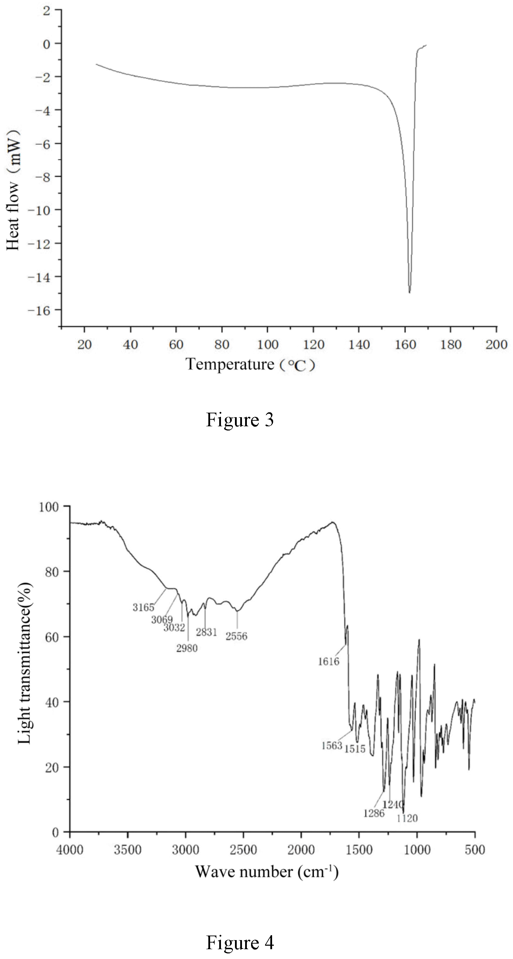

In some embodiments, the differential scanning calorimetry (DSC) thermogram of the curcumin-L-carnitine cocrystal has a characteristic endothermic peak at 161 ± 2 °C; preferably, the curcumin-L-carnitine cocrystal has a DSC thermogram substantially as shown in Figure 3.

In some embodiments, the infrared spectrum of the curcumin-L-carnitine cocrystal has characteristic peaks at 3032 cm⁻¹ ± 2 cm⁻¹, 2980 cm⁻¹ ± 2 cm⁻¹, and 2556 cm⁻¹ ± 2 cm⁻¹; in particular, it further has characteristic peaks at 3069 cm⁻¹ ± 2 cm⁻¹, 2831 cm⁻¹ ± 2 cm⁻¹, 1563 cm⁻¹ ± 2 cm⁻¹, 1515 cm⁻¹ ± 2 cm⁻¹, 1286 cm⁻¹ ± 2 cm⁻¹, 1240 cm⁻¹ ± 2 cm⁻¹, and 1120 cm⁻¹ ± 2 cm⁻¹; preferably, it has an infrared spectrum substantially as shown in Figure 4.

In a second aspect, the present invention provides a method for preparing the curcumin-L-carnitine cocrystal, wherein the method is selected from one of the following methods:
Method 1: recrystallizing curcumin and L-carnitine in a stoichiometric ratio of 1:1 to 1:2 in a solvent, and isolating and drying the precipitate to obtain the curcumin-L-carnitine cocrystal;
Method 2: ball-milling curcumin and L-carnitine in a stoichiometric ratio of 1:1 in a solvent, and drying the resulting crystals to obtain the curcumin-L-carnitine cocrystal.

In Method 1 and Method 2 described above, the solvent is selected from solvents that have a certain solubility for the raw materials and do not cause their degradation. Preferably, the solvent is independently one or more selected from the group consisting of water, alcohols, ketones, esters, alkanes, aromatic hydrocarbons, and halogenated alkanes; more preferably, the solvent is independently one or more selected from the group consisting of methanol, ethanol, isopropanol, ethyl acetate, isopropyl acetate, acetone, methyl ethyl ketone, methyl tert-butyl ether, n-hexane, and n-heptane.

According to Method 1 described above:
In some embodiments, the ratio of the total mass of curcumin and L-carnitine to the volume of the solvent is 1 g : (3-20) mL, preferably 1 g : (4-15) mL.
In some embodiments, the recrystallization temperature is 10 to 70 °C, preferably 20 to 40 °C, for example 20 °C, 25 °C, 30 °C, 35 °C, or 40 °C; the recrystallization time is 1 to 36 h, preferably 10 to 24 h, for example 6 h, 10 h, 12 h, 16 h, 18 h, 20 h, or 24 h.
In some embodiments, the separation of crystals can be carried out by any separation method that does not adversely affect the cocrystal, for example, filtration or centrifugation may be used to separate the crystals from the solvent.

According to Method 2 described above:
In some embodiments, the ratio of the total mass of curcumin and L-carnitine to the volume of the solvent is 1 g : (0.1-5) mL, preferably 1 g : (0.5-2) mL.
In some embodiments, the ball milling time is 10 min to 180 min, preferably 30 min to 60 min, for example 30 min, 40 min, 50 min, or 60 min.
In both Method 1 and Method 2 described above, the drying of the crystals can be carried out by any drying method that does not adversely affect the cocrystal, such as vacuum drying, fluidized bed drying, or forced air drying, preferably vacuum drying. The drying time can be 3 to 36 h, preferably 6 to 18 h, for example 6 h, 8 h, 12 h, 16 h, 18 h, 22 h, 24 h, 28 h, 32 h, or 36 h.

This invention relates to a preparation method that is simple to operate, with an easily controllable crystallization process, high crystallinity, and good reproducibility, enabling stable acquisition of curcumin-L-carnitine cocrystals.

In a third aspect, the present invention provides a curcumin product comprising the curcumin-L-carnitine cocrystal, wherein the product is selected from health products, foods, cosmetics, pharmaceuticals, pharmaceutical excipients, and feeds.

In a fourth aspect, the present invention provides a use of the curcumin-L-carnitine cocrystal in the preparation of a curcumin product, wherein the product is selected from health products, foods, cosmetics, pharmaceuticals, pharmaceutical excipients, and feeds.

The product may also contain other suitable raw materials as required, for example, foods may comprise food base materials and edible food additives acceptable for food use, such as sweeteners, flavoring agents, preservatives, fragrances, colorants, etc.; cosmetics may comprise cosmetic base materials and additives acceptable for cosmetic use, such as solvents, fragrances, preservatives, essences, colorants, etc.; pharmaceuticals may comprise pharmaceutically active ingredients and pharmaceutically acceptable excipients, such as carriers, diluents, adjuvants, colorants, etc.; feeds may comprise feed base materials, such as soybean meal, hay, etc., and feed adjuncts acceptable for feed use, such as sweeteners, flavoring agents, preservatives, fragrances, colorants, etc., but the present invention is not limited thereto.

The above products are prepared by adding the curcumin-L-carnitine cocrystal of the present invention. Except for the addition of the curcumin-L-carnitine cocrystal of the present invention, the preparation methods for such products can follow conventional procedures.

The present invention has been described in detail above, but the above embodiments are merely illustrative in nature and are not intended to limit the present invention. Furthermore, this document is not limited by any theory set forth in the prior art, the summary of the invention, or the following examples.

Unless explicitly stated otherwise, all numerical ranges throughout the application document include any sub-ranges and any numerical values increasing by the smallest unit of the given value. Unless explicitly stated otherwise, all numerical values throughout the application document represent approximate measures or limits that include minor deviations from the given value, as well as ranges that are approximately at the recited value and those that are exactly at the recited value. Except for the working examples provided at the end of the detailed description, all numerical values for parameters (e.g., amounts or conditions) in this application document, including the appended claims, are in all cases to be understood as modified by the term "about," regardless of whether "about" actually appears before the numerical value. "About" indicates that the stated value allows for some slight imprecision (a close approximation to the value; approximately or reasonably close to the value; roughly). If the imprecision provided by "about" is not understood in this ordinary sense in the art, then "about" as used herein refers to at least variations that may result from ordinary methods of measurement and use of these parameters. For example, "about" may include variations of less than or equal to 10%, less than or equal to 5%, less than or equal to 4%, less than or equal to 3%, less than or equal to 2%, less than or equal to 1%, or less than or equal to 0.5%, and in some aspects, less than or equal to 0.1%.

Unless explicitly stated otherwise, the terms "comprising," "including," "having," "containing," or any other similar terms throughout the application document are open-ended terms, meaning that a cocrystal or product may include, in addition to those elements listed herein, other elements not explicitly listed but typically inherent to the cocrystal or product. Furthermore, in this document, the terms "comprising," "including," "having," and "containing" should be interpreted as specifically disclosing and simultaneously encompassing the closed or semi-closed transitional phrases such as "consisting of" and "consisting essentially of." "Consisting essentially of" means that the listed elements constitute more than 95%, more than 97%, or in some aspects, more than 99% of the cocrystal or product.

### Beneficial Effects

(1) The present invention provides a stable curcumin-L-carnitine cocrystal. Compared to curcumin crystals themselves, this cocrystal increases the solubility and dissolution performance of curcumin, significantly improving its absorption and bioavailability. This can further broaden the application range of curcumin, enhance its effectiveness, and reduce its dosage and usage cost, thus having strong practical application value.
(2) The preparation method of the curcumin-L-carnitine cocrystal of the present invention is simple, has good reproducibility, and offers advantages such as low cost, environmental friendliness, and easy control.

### Brief Description of Drawings

Figure 1 is the single crystal structure diagram of the curcumin-L-carnitine cocrystal prepared in Example 1 of the present invention;
Figure 2 is the X-ray powder diffraction (XRPD) pattern of the curcumin-L-carnitine cocrystal prepared in Example 1 of the present invention;
Figure 3 is the differential scanning calorimetry (DSC) thermogram of the curcumin-L-carnitine cocrystal prepared in Example 1 of the present invention;
Figure 4 is the infrared (IR) spectrum of the curcumin-L-carnitine cocrystal prepared in Example 1 of the present invention;
Figure 5 is the infrared (IR) spectrum of commercially available curcumin crystals used in Example 1 of the present invention;
Figure 6 is a comparison of dissolution curves of commercially available curcumin crystals and the curcumin-L-carnitine cocrystal prepared in Example 3 in pH 2.0 buffer solution in the Test Example;
Figure 7 is a comparison of dissolution curves of commercially available curcumin crystals and the curcumin-L-carnitine cocrystal prepared in Example 3 in pH 4.5 buffer solution in the Test Example;
Figure 8 is a comparison of plasma concentration curves of commercially available curcumin crystals and the curcumin-L-carnitine cocrystal prepared in Example 3 in Test Example 2.

### Detailed Description

In order to make the objectives, technical solutions, and advantages of the present invention clearer, the following further details the present invention with reference to the accompanying drawings and examples. It should be understood that the specific embodiments described herein are only for explaining the present invention and are not intended to limit the present invention.

The present invention uses L-carnitine as a ligand to form a stable cocrystal with curcumin. It can be seen from the following single crystal structures that the interactions and arrangements between curcumin molecules in the curcumin crystal and in the curcumin-L-carnitine cocrystal are completely different. In the curcumin crystal, curcumin molecules form a two-dimensional network structure through hydrogen bonds between phenolic hydroxyl groups and carbonyl groups; in the curcumin-L-carnitine cocrystal, curcumin molecules use L-carnitine molecules as a bridge, forming a one-dimensional chain structure through hydrogen bonds between phenolic hydroxyl groups and carboxylate groups, as well as between hydroxyl groups and carboxylate groups.

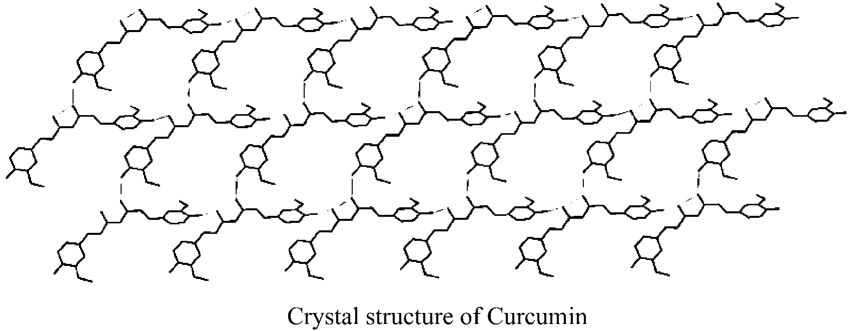

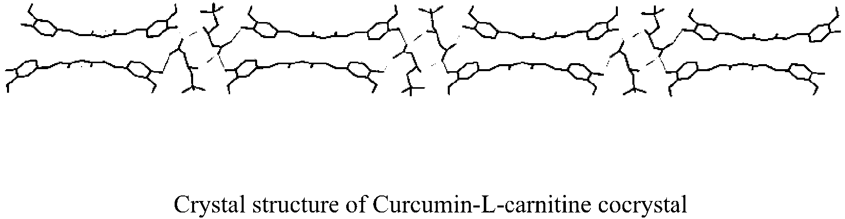

In the present invention, X-ray powder diffraction patterns were obtained using a Bruker D8 Advance X-ray diffractometer. The instrument uses Cu-Kα radiation (λ = 1.54056 Å), with a scan range over the 20 interval from 3° to 40°, and a scan speed of 5° /minute.

X-ray single crystal data was collected by a Bruker D8 Venture X-ray single crystal diffractometer, equipped with a Mo-Kα X-ray radiation source (λ = 0.71073 Å). The test temperature was 170 K, voltage was 50 kV, current was 30 mA.

Differential scanning calorimetry was performed using a TA DSC Q2000 device with a heating rate of 10 K/min.

Fourier transform infrared spectroscopy was performed using a Thermo Scientific Nicolet 6700.

Ball milling was performed using a Jingxin JX-2G planetary ball mill.

Liquid chromatography was performed using an Agilent 1260 Infinity HPLC.

The reagents and source information used in the following examples are as follows:
L-carnitine: Purity 99%, purchased from Aladdin Reagent Co., Ltd.;
Curcumin (crystals): Purity 98%, purchased from Aladdin Reagent Co., Ltd.;
Ethanol: Purity 99%, purchased from Sinopharm Chemical Reagent Co., Ltd.;
Ethyl acetate: Purity 99%, purchased from Sinopharm Chemical Reagent Co., Ltd.;
Methanol: Purity 99%, purchased from Sinopharm Chemical Reagent Co., Ltd.

### Example 1

1.61g of L-carnitine and 3.69g of curcumin were added to 60 mL of ethanol, stirred as a suspension at 30°C for 12 hours, filtered to obtain a red solid, and dried under vacuum overnight to obtain the curcumin-L-carnitine cocrystal.

This curcumin-L-carnitine cocrystal, along with raw curcumin crystals and L-carnitine, were characterized by X-ray powder diffraction (XRPD). The curcumin-L-carnitine cocrystal was also analyzed by differential scanning calorimetry (DSC) and infrared (IR) spectroscopy. The results are shown in Tables 1-3, and Figures 2-5.

As shown in Tables 1-3 and Figure 2, the curcumin-L-carnitine cocrystal has a completely different XRPD pattern compared to curcumin and L-carnitine themselves. The curcumin-L-carnitine cocrystal has characteristic peaks at 2θ angles of 5.0° ± 0.2°, 6.4° ± 0.2°, 13.1° ± 0.2°, 16.8° ± 0.2°, 20.3° ± 0.2°, and 23.9° ± 0.2°.

As shown in Figures 4-5, the curcumin-L-carnitine cocrystal has a completely different IR spectrum compared to curcumin itself. In the IR spectrum of the curcumin-L-carnitine cocrystal, the characteristic infrared peak of the curcumin phenolic hydroxyl group is redshifted from 3508 cm⁻¹ to 3165 cm⁻¹, indicating a strong hydrogen bonding interaction between curcumin and L-carnitine in the cocrystal.

**Table 1. XRPD data for curcumin-L-carnitine cocrystal**

| **Peak No.** | **Diffraction Angle (20, °)** | **Intensity (I/I₀, %)** | **Peak No.** | **Diffraction Angle (2θ, °)** | **Intensity (I/I₀, %)** |
|---|---|---|---|---|---|
| 1 | 5.026 | 14.4 | 14 | 23.934 | 71.3 |
| 2 | 6.389 | 5.3 | 15 | 24.857 | 4 |
| 3 | 8.995 | 5.7 | 16 | 25.419 | 18.5 |
| 4 | 13.126 | 8.7 | 17 | 25.939 | 10.6 |
| 5 | 15.011 | 20.2 | 18 | 26.463 | 6.7 |
| 6 | 15.714 | 35.7 | 19 | 27.865 | 36.8 |
| 7 | 16.776 | 55.1 | 20 | 29.207 | 19.5 |
| 8 | 18.22 | 61.1 | 21 | 29.787 | 3.1 |
| 9 | 18.62 | 37.3 | 22 | 30.327 | 3 |
| 10 | 20.305 | 100 | 23 | 31.394 | 6.1 |
| 11 | 21.349 | 5.4 | 24 | 32.016 | 6.9 |
| 12 | 22.07 | 53.4 | 25 | 35.044 | 6.2 |
| 13 | 22.591 | 50.8 | 26 | 35.564 | 3.2 |

**Table 2. XRPD data for commercially available Curcumin**

| **Peak No.** | **Diffraction Angle (20, °)** | **Intensity (I/I₀, %)** | **Peak No.** | **Diffraction Angle (20, °)** | **Intensity (I/I₀, %)** |
|---|---|---|---|---|---|
| 1 | 7.911 | 25.5 | 15 | 21.109 | 43.8 |
| 2 | 8.833 | 25.7 | 16 | 21.87 | 3.7 |
| 3 | 12.245 | 6.1 | 17 | 22.251 | 2.5 |
| 4 | 13.966 | 7.4 | 18 | 22.772 | 4.9 |
| 5 | 14.47 | 17.5 | 19 | 23.271 | 17.6 |
| 6 | 15.15 | 3.5 | 20 | 23.675 | 15.2 |
| 7 | 15.557 | 2.7 | 21 | 24.697 | 13.6 |
| 8 | 15.892 | 34 | 22 | 25.158 | 5.8 |
| 9 | 16.355 | 3.9 | 23 | 25.717 | 9.9 |
| 10 | 17.198 | 100 | 24 | 25.996 | 2.5 |
| 11 | 17.778 | 6 | 25 | 26.22 | 4 |
| 12 | 18.138 | 11.2 | 26 | 26.68 | 13.9 |
| 13 | 18.862 | 10.1 | 27 | 27.004 | 3.4 |
| 14 | 19.442 | 8.9 | | | |

**Table 3. XRPD data for L-carnitine**

| **Peak No.** | **Diffraction Angle (20, °)** | **Intensity (I/I₀, %)** | **Peak No.** | **Diffraction Angle (20, °)** | **Intensity (I/I₀, %)** |
|---|---|---|---|---|---|
| 1 | 8.922 | 23.4 | 9 | 22.553 | 26.5 |
| 2 | 9.324 | 5.3 | 10 | 23.09 | 11.1 |
| 3 | 15.945 | 5.9 | 11 | 23.247 | 5.7 |
| 4 | 17.874 | 33.5 | 12 | 23.663 | 11.1 |
| 5 | 18.659 | 16.1 | 13 | 23.930 | 7.9 |
| 6 | 18.852 | 22.1 | 14 | 25.651 | 8.8 |
| 7 | 20.151 | 51.7 | 15 | 26.052 | 5.9 |
| 8 | 20.2 | 100.0 | 16 | 26.928 | 14.2 |

### Example 2

1.61 g of L-carnitine and 3.69 g of curcumin were added to 60 mL of ethyl acetate, stirred as a suspension at 30°C for 12 hours, filtered to obtain a red solid, and dried under vacuum overnight to obtain the curcumin-L-carnitine cocrystal.

This cocrystal was characterized by X-ray powder diffraction (XRPD), differential scanning calorimetry (DSC), and infrared (IR) spectroscopy. The results were essentially consistent with Figures 2 to 4.

### Example 3

0.161 g of L-carnitine and 0.369 g of curcumin were added to a ball mill jar, 1 mL of ethanol was added, and the mixture was ball-milled at room temperature for 0.5 hours. The solid was dried in a vacuum drying oven at room temperature for 12 hours to obtain the curcumin-L-carnitine cocrystal.

This cocrystal was characterized by X-ray powder diffraction (XRPD), differential scanning calorimetry (DSC), and infrared (IR) spectroscopy. The results were essentially consistent with Figures 2 to 4.

### Example 4

0.161 g of L-carnitine and 0.369 g of curcumin were added to a ball mill jar, 1 mL of methanol was added, and the mixture was ball-milled at room temperature for 0.5 hours. The solid was dried in a vacuum drying oven at room temperature for 12 hours to obtain the curcumin-L-carnitine cocrystal.

This cocrystal was characterized by X-ray powder diffraction (XRPD), differential scanning calorimetry (DSC), and infrared (IR) spectroscopy. The results were essentially consistent with Figures 2 to 4.

### Comparative Example 1

0.131 g of leucine and 0.369 g of curcumin were added to a ball mill jar, 1 mL of methanol or ethanol was added, and the mixture was ball-milled for 0.5 hours. The solid was dried in a vacuum drying oven at room temperature for 12 hours to obtain a yellow powder.

This powder was tested by X-ray powder diffraction (XRPD) and no cocrystal was found.

### Comparative Example 2

0.131 g of isoleucine and 0.369 g of curcumin were added to a ball mill jar, 1 mL of methanol or ethanol was added, and the mixture was ball-milled for 0.5 hours. The solid was dried in a vacuum drying oven at room temperature for 12 hours to obtain a yellow powder.

This powder was tested by X-ray powder diffraction (XRPD) and no cocrystal was found.

### Comparative Example 3

0.139 g of methionine and 0.369 g of curcumin were added to a ball mill jar, 1 mL of methanol or ethanol was added, and the mixture was ball-milled for 0.5 hours. The solid was dried in a vacuum drying oven at room temperature for 12 hours to obtain a yellow powder.

This powder was tested by X-ray powder diffraction (XRPD) and no cocrystal was found.

### Comparative Example 4

0.119 g of threonine and 0.369 g of curcumin were added to a ball mill jar, 1 mL of methanol or ethanol was added, and the mixture was ball-milled for 0.5 hours. The solid was dried in a vacuum drying oven at room temperature for 12 hours to obtain a yellow powder.

This powder was tested by X-ray powder diffraction (XRPD) and no cocrystal was found.

### Test Example 1: Dissolution Rate Test

A person skilled in the art can demonstrate that the curcumin-L-carnitine cocrystal described herein has a significantly improved dissolution rate compared to curcumin crystals themselves. Representative studies were conducted using commercially available curcumin crystals and the curcumin-L-carnitine cocrystal obtained in Example 3.

Comparison of powder dissolution differences was performed on commercially available curcumin and the curcumin-L-carnitine cocrystal obtained in Example 3.

Powder dissolution experiments were conducted in pH 2.0 and pH 4.5 buffer solutions (containing 0.5% Tween 80) at a temperature of 37°C and a rotation speed of 50 rpm.

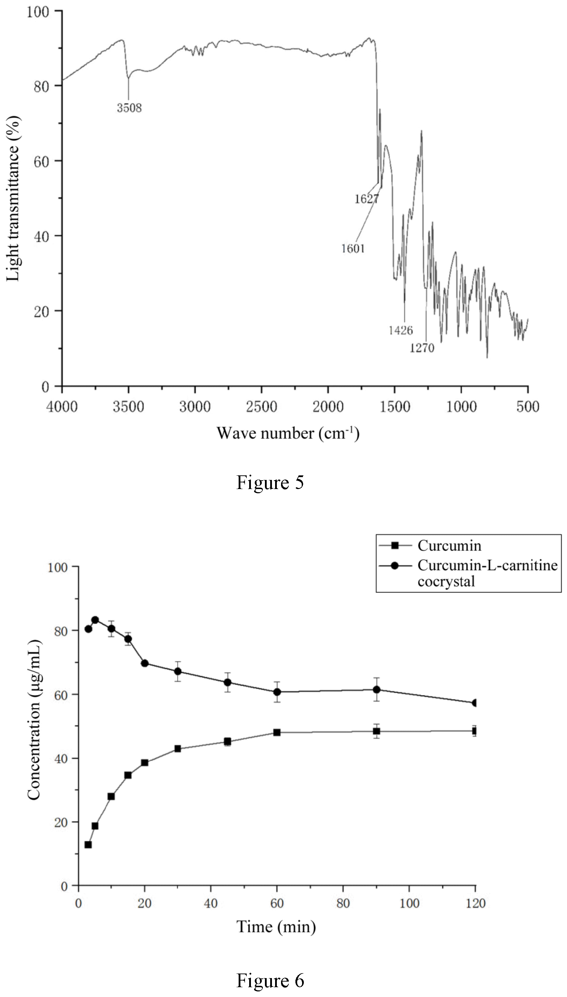

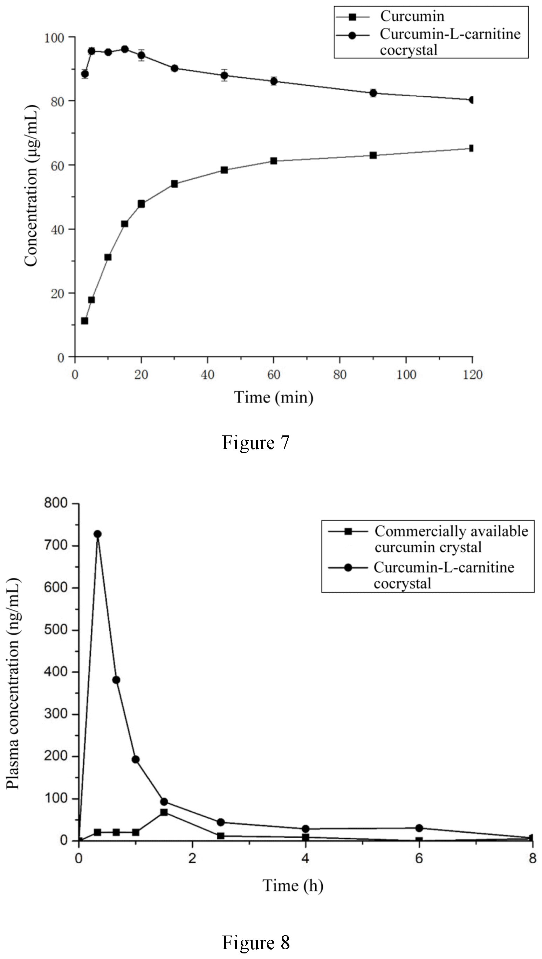

The commercially available curcumin crystals and the powder of the curcumin-L-carnitine cocrystal from Example 3 were sieved through a 100-mesh screen to eliminate the effect of particle size on dissolution. Samples equivalent to 10 mg of curcumin were placed into the sample vessels of the dissolution apparatus, stirred, and samples were taken at 3, 5, 10, 15, 20, 30, 45, 60, 90, and 120 minutes. Concentrations were determined using HPLC. The results are shown in Figures 6 and 7. In the pH 2.0 buffer solution, the dissolution of the cocrystal at 3 minutes was about 7 times that of curcumin itself. In the pH 4.5 buffer solution, the dissolution of the cocrystal at 3 minutes was about 8 times that of curcumin itself.

As shown by the above results, compared with conventional commercially available curcumin crystals, the curcumin cocrystal disclosed in the present invention exhibits superior solubility and dissolution rate.

### Test Example 2: Bioavailability Test

A person skilled in the art can demonstrate that the curcumin-L-carnitine cocrystal described herein has significantly improved bioavailability compared to commercially available curcumin crystals. Representative studies were conducted using commercially available curcumin crystals and the curcumin-L-carnitine cocrystal obtained in Example 3.

Comparison of bioavailability differences was performed on commercially available curcumin and the curcumin-L-carnitine cocrystal obtained in Example 3.

Male SD rats (weighing 200-300 g) kept under good feeding conditions were used for the experiment. A total of 12 rats were used, evenly divided into 2 groups of 6 rats each. Commercially available curcumin crystals and the cocrystal obtained in Example 3 were uniformly dispersed in soybean oil and administered intragastrically as a suspension. The dosage was 200 mg/kg based on curcumin content. Blood samples (1 mL per time point) were collected from the orbital venous plexus at 20 minutes, 40 minutes, 1 hour, 1.5 hours, 2.5 hours, 4 hours, 6 hours, and 8 hours after administration. Plasma was separated by centrifugation within 0.5 hours of blood collection at 10,000 rpm for 5 minutes. 100 µL of plasma was precisely transferred, added with 50 µL of enzyme buffer solution, incubated at 37°C for 60 min, then added with 0.45 mL of methanol (containing 0.2% acetic acid), vortexed for 10 min, centrifuged at 14000 rpm for 3 min, and the supernatant was collected for HPLC analysis. The results are shown in Table 4 and Figure 8.

**Table 4. Pharmacokinetic parameters for commercially available curcumin crystals and curcumin-L-carnitine cocrystal.**

| Formulation | Tₘₐₓ (min) | Cₘₐₓ (ng/mL) | AUC₀₋₈ₕ (ng·h/mL) |
|---|---|---|---|
| Commercially available curcumin crystals | 90 | 67.9 | 109.9 |
| Curcumin-L-carnitine cocrystal | 20 | 727.8 | 692.5 |

As shown by the above results, compared with conventional commercially available curcumin crystals, the curcumin-L-carnitine cocrystal of the present invention exhibits superior bioavailability. The maximum plasma concentration of curcumin in rats administered with the cocrystal reached 10.7 times that of rats administered with the commercially available curcumin crystals.

## Claims

1. A cocrystal of curcumin and L-carnitine, wherein the stoichiometric ratio of curcumin to L-carnitine in the cocrystal is 1:1.

2. The cocrystal of curcumin and L-carnitine according to claim 1, **characterized in that** the cocrystal of curcumin and L-carnitine belongs to the monoclinic system, and has unit cell parameters of a = 17.1370(11) Å, b = 5.6406(4) Å, c = 27.393(2) Å, elongs to the monoclinic system, a

3. The cocrystal of curcumin and L-carnitine according to claim 1, **characterized in that** the X-ray powder diffraction pattern of the cocrystal of curcumin and L-carnitine has characteristic peaks at 2θ angles of 5.0°±0.2°, 6.4°±0.2°, 13.1°±0.2°, 16.8°±0.2°, 20.3° ±0.2°, and 23.9°±0.2°;
preferably, the X-ray powder diffraction pattern of the cocrystal of curcumin and L-carnitine further has characteristic peaks at 2θ angles of 18.2°±0.2°, 18.6°±0.2°, 22.1° ±0.2°, and 22.6°±0.2°;
more preferably, the cocrystal of curcumin and L-carnitine has an X-ray powder diffraction pattern substantially as shown in Figure 2.

4. The cocrystal of curcumin and L-carnitine according to claim 1, **characterized in that** the differential scanning calorimetry thermogram of the cocrystal of curcumin and L-carnitine has a characteristic endothermic peak at 161±2 °C;
preferably, the cocrystal of curcumin and L-carnitine has a differential scanning calorimetry thermogram substantially as shown in Figure 3.

5. The cocrystal of curcumin and L-carnitine according to claim 1, **characterized in that** the infrared spectrum of the cocrystal of curcumin and L-carnitine has characteristic peaks at 3032 cm⁻¹±2 cm⁻¹, 2980 cm⁻¹±2 cm⁻¹, and 2556 cm⁻¹±2 cm⁻¹;
specifically, the infrared spectrum of the cocrystal of curcumin and L-carnitine further has characteristic peaks at 3069 cm⁻¹±2 cm⁻¹, 2831 cm⁻¹±2 cm⁻¹, 1563 cm⁻¹±2 cm⁻¹, 1515 cm⁻¹±2 cm⁻¹, 1286 cm⁻¹±2 cm⁻¹, 1240 cm⁻¹±2 cm⁻¹, and 1120 cm⁻¹±2 cm⁻¹,
preferably, the cocrystal of curcumin and L-carnitine has an infrared spectrum substantially as shown in Figure 4.

6. A method for preparing the cocrystal of curcumin and L-carnitine according to any one of claims 1-5, wherein the method is selected from one of the following methods:
Method one: recrystallizing curcumin and L-carnitine in a stoichiometric ratio of 1:1 to 1:2 in a solvent, separating and drying the precipitate to obtain the cocrystal of curcumin and L-carnitine;
Method two: ball-milling curcumin and L-carnitine in a stoichiometric ratio of 1:1 in a solvent, drying the obtained crystals to obtain the cocrystal of curcumin and L-carnitine.

7. The method for preparing the cocrystal of curcumin and L-carnitine according to claim 6, wherein in the above Method one and Method two, the solvent is independently one or more selected from the group consisting of water, alcohols, ketones, esters, alkanes, aromatic hydrocarbons, and halogenated alkanes;
preferably, the solvent is independently one or more selected from the group consisting of methanol, ethanol, isopropanol, ethyl acetate, isopropyl acetate, acetone, methyl ethyl ketone, methyl tert-butyl ether, n-hexane, and n-heptane; and/or
the method for drying the crystals is vacuum drying, fluidized bed drying, or forced air drying, preferably vacuum drying, with a drying time of 3 to 36 hours, preferably 6 to 18 hours.

8. The method for preparing the cocrystal of curcumin and L-carnitine according to claim 6, **characterized in that**,
in Method one:
the ratio of the total mass of curcumin and L-carnitine to the volume of solvent is 1g : (3-20) mL, preferably 1g : (4-15) mL; and/or
the recrystallization temperature is 10 to 70 ° C, preferably 20 to 40 ° C; the recrystallization time is 1 to 36 hours, preferably 10 to 24 hours;
in Method two:
the ratio of the total mass of curcumin and L-carnitine to the volume of solvent is 1g : (0.1-5) mL, preferably 1g : (0.5-2) mL.

9. A curcumin product comprising the cocrystal of curcumin and L-carnitine according to any one of claims 1-5 or the cocrystal of curcumin and L-carnitine prepared by the method according to any one of claims 6-8, wherein the product is selected from health products, foods, cosmetics, pharmaceuticals, pharmaceutical excipients, and feeds.

10. A use of the cocrystal of curcumin and L-carnitine according to any one of claims 1-5, or the cocrystal of curcumin and L-carnitine prepared by the method according to any one of claims 6-8, in the preparation of curcumin products, wherein the product is selected from health products, foods, cosmetics, pharmaceuticals, pharmaceutical excipients, and feeds.
